# EUROPÄISCHE PATENTSCHRIFT

(11) **EP 0 893 441 B1**
(45) Veröffentlichungstag und Bekanntmachung des Hinweises auf die Patenterteilung: **02.01.2003**
(21) Anmeldenummer: 98112969.5
(22) Anmeldetag: 13.07.1998
(51) Int. Cl.: C07D 213/61, C07D 213/38

(54) **Verfahren zur Herstellung von Pyridylmethylisothiocyanaten**
Process for the production of pyridyl methylisothiocyanate
Procédé pour la préparation de méthyleisothiocyanate de pyridyle

(30) Priorität: 24.07.1997 DE 19731782
(43) Veröffentlichungstag der Anmeldung: 27.01.1999
(73) Patentinhaber: Bayer CropScience AG, 40789 Monheim (DE)
(72) Erfinder: Wagner, Klaus Dr., 51065 Köln (DE); Lantzsch, Reinhard Dr., 41225 Wuppertal (DE)

(56) Entgegenhaltungen:
- EP-A- 0 302 389
- US-A- 4 129 656
- DATABASE XFIRE BEILSTEIN Reaction ID2437787 and 2626662, 1980 RAHMAN M F: ""Synthesis of 4-substituted thiosemicarbazones of 3-methyl-4-phenylpyridine-2-carboxaldehyde as antitumor agents" XP002077168 & RAHMAN M F: "Synthesis of 4-substituted thiosemicarbazones of 3-methyl-4-phenylpyridine-2-carboxaldehyde as antitumor agents" INDIAN J CHEM., SECT B, Bd. 80, Nr. 19b, 1980, INDIA, Seiten 828-830, XP002077167

## Beschreibung

Die vorliegende Erfindung betrifft ein neues Verfahren zur Herstellung von Pyridylmethylisothiocyanaten sowie neue Zwischenprodukte.

Es ist bekannt, daß man Pyridylmethylisothiocyanate erhält, wenn man Pyridylmethylamine der Formel (A) mit Kohlenstoffdisulfid und z.B. Ethyl-chlorcarbonat in Gegenwart einer Base, wie beispielsweise Natriumhydroxid gemäß dem folgenden Reaktionsschema umsetzt: 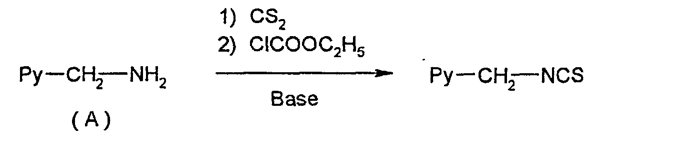 Py = gegebenenfalls substituiertes Pyridyl, wie z.B. 2-Chlor-5-pyridyl
(vgl. hierzu EP-A 0 302 389).

Dieses Verfahren hat jedoch den Nachteil, daß Kohlenstoffdisulfid als Reaktionspartner eingesetzt wird. Außerdem fällt Kohlenoxisulfid als Nebenprodukt an.

Ein ähnliches Verfahren erfordert neben dem Einsatz von Kohlenstoffdisulfid (CS₂) die Verwendung von dem explosiven Natriumchlorit (NaClO₂) (vgl. Ind. J. Chem., Sect. B 1980, 19, 828-830).

Es ist weiterhin bekannt, dass man unsubstituierte Pyridylmethylisothiocyanate und unsubstituierte Pyridylmethyldithiocarbamate als Vorstufen für Thiazolidin-Derivate mit saluretischer Wirkung verwenden kann (vgl. US 4,129,656).

Es wurde nun gefunden, daß man Pyridylmethylisothiocyanate der Formel (I) 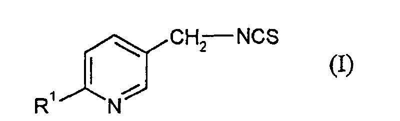 in welcher
- R¹: für Halogen oder C₁-C₄-Alkyl steht
in guten Ausbeuten und hoher Reinheit erhält, wenn man in einer ersten Stufe Amine der Formel (II) 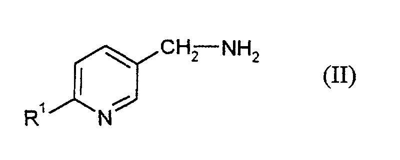 in welcher
- R¹: die oben angegebene Bedeutung hat,
mit Xanthogenaten der Formel (III)

R²O―CS―S^{⊖}M^{⊕} (III)

in welcher
- R²: für C₁-C₄-Alkyl, vorzugsweise Methyl oder Ethyl steht und
- M: für ein Alkalimetall, vorzugsweise Natrium und Kalium oder für Ammonium steht,
gegebenenfalls in Gegenwart eines Verdünnungsmittels umsetzt und
in einer zweiten Stufe die so erhaltenen Dithiocarbamate der Formel (IV) 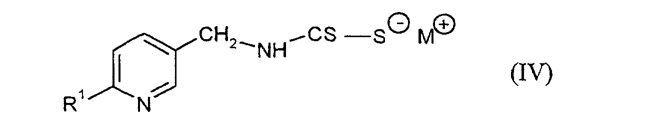 in welcher
- R¹ und M: die oben angegebene Bedeutung haben,
gegebenenfalls in Gegenwart eines Verdünnungsmittels zu den Isothiocyanaten der Formel (I) oxidiert.

Die erfindungsgemäß herstellbaren Pyridylmethylisothiocyanate sind durch die Formel (I) allgemein definiert. In dieser Formel steht R¹ vorzugsweise für Chlor oder Methyl.

Überraschenderweise können nach dem erfindungsgemäßen Verfahren die Pyridylmethylisothiocyanate der Formel (I) in guten Ausbeuten und in hoher Reinheit erhalten werden, obwohl in den Xanthogenaten der Formel (III) der schwefelhaltige Rest MSH die bessere Abgangsgruppe gegenüber dem Alkoholrest R²OH darstellt und somit eingentlich der folgende Reaktionsablauf zu erwarten gewesen wäre:

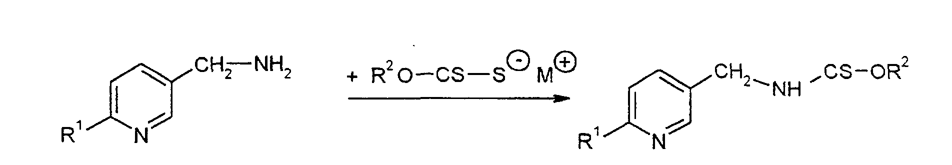

Die erfindungsgemäße Umsetzung hat den Vorteil, daß auf Kohlenstoffdisulfid als Reaktionspartner verzichtet werden kann. Außerdem handelt es sich um eine einfache Reaktionsdurchführung (keine exotherme Reaktion), die ohne Nebenreaktionen verläuft.

Verwendet man beispielsweise 5-Aminomethyl-2-chlorpyridin und Kaliumethylxanthogenat als Ausgangsstoffe sowie Chlorlauge (NaOCl) als Oxidationsmittel, so kann der Reaktionsablauf des erfindungsgemäßen Verfahrens durch das folgende Formelschema skizziert werden:

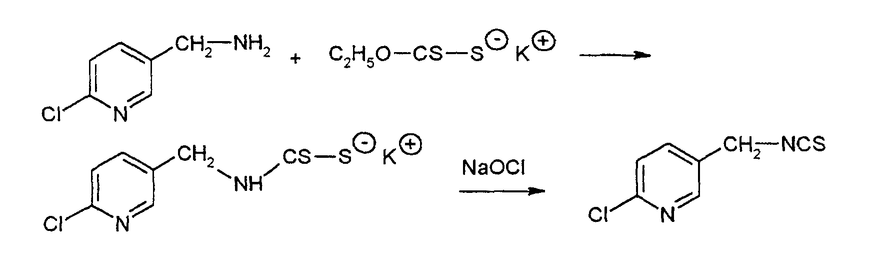

Die beim erfindungsgemäßen Verfahren in der ersten Stufe als Ausgangsstoffe zu verwendenden Amine der Formel (II) sind bekannt (vgl. z.B. EP-A 0 391 205 oder US-P 4 499 097) und/oder in allgemein bekannter Art und Weise erhältlich.

Die außerdem beim erfindungsgemäßen Verfahren in der ersten Stufe als Ausgangsstoffe zu verwendenden Xanthogenate der Formel (III) sind allgemein bekannte Verbindungen.

Die beim erfindungsgemäßen Verfahren in der ersten Stufe erhältlichen Dithiocarbamate sind durch die Formel (IV) allgemein definiert. In dieser Formel stehen R¹ vorzugsweise für Chlor oder Methyl und M vorzugsweise für Natrium, Kalium oder Ammonium.

Die Dithiocarbamate der Formel (IV) sind neu und ebenfalls Gegenstand der vorliegenden Anmeldung.

Als Oxidationsmittel kommen für die zweite Stufe des erfindungsgemäßen Verfahrens vorzugsweise infrage: Chlorlauge (Natrium- oder Kaliumhypochlorit) und Kupfer-II-Verbindungen, wie Kupfersulfat.

Als Verdünnungsmittel kommen für die erste Stufe des erfindungsgemäßen Verfahrens übliche organische Lösungsmittel infrage. Hierzu gehören vorzugsweise Kohlenwasserstoffe, wie Toluol oder Xylol; Ether wie Methyl-tert.Butyl-ether, Methyltert. Amyl-ether, 1,2-Dimethoxiethan, 1,2-Diethoxiethan, Tetrahydrofuran oder Dioxan; Alkohole, wie Methanol, Ethanol, n-Propanol, i-Propanol, n-, i-, s- oder t-Butanol; Nitrile, wie Acetonitril, Propionitril oder Butyronitril; sowie Amide, wie Dimethylformamid.

Die Reaktionstemperaturen können bei der Durchführung der ersten Stufe des erfindungsgemäßen Verfahrens in einem größeren Bereich variiert werden. Im allgemeinen arbeitet man bei Temperaturen zwischen 30°C und 150°C, vorzugsweise bei Temperaturen zwischen 50°C und 120°C.

Bei der Durchführung der ersten Stufe des erfindungsgemäßen Verfahrens arbeitet man vorzugsweise in äquimolaren Mengen. Es ist aber auch möglich, das preiswertere Xanthogenat der Formel (III) in größerem Überschuß einzusetzen, vorzugsweise 100 %.

Reaktionsdurchführung, Aufarbeitung und Isolierung der neuen Dithiocarbamate der Formel (IV) erfolgen in allgemein üblicher Art und Weise (vgl. auch das Herstellungsbeispiel).

Als Verdünnungsmittel kommen für die Oxidation gemäß der zweiten Stufe des erfindungsgemäßen Verfahrens vorzugsweise Wasser und ein zweites inertes, mit Wasser wenig mischbares Lösungsmittel infrage, wie beispielsweise Kohlenwasserstoffe, Chlorkohlenwasserstoffe, Ether; Nitrile, Ketone oder Amide.

Die Reaktionstemperaturen können bei der Durchführung der zweiten Stufe des erfindungsgemäßen Verfahrens in einem größeren Bereich variiert werden. Im allgemeinen arbeitet man bei Temperaturen zwischen -30°C und 50°C, vorzugsweise bei Temperaturen zwischen -10°C und 10°C.

Bei der Durchführung der zweiten Stufe des erfindungsgemäßen Verfahrens setzt man auf 1 Mol Dithiocarbamat im allgemeinen 4 bis 5 Mol, vorzugsweise 4 bis 4,5 Mol an Oxidationsmittel ein.

Reaktionsdurchführung, Aufarbeitung und Isolierung der Pyridylmethylisothiocyanate der Formel (I) erfolgen in allgemein üblicher Art und Weise (vgl. auch das Herstellungsbeispiel).

Die nach dem erfindungsgemäßen Verfahren herzustellenden Pyridylmethylisothiocyanate der Formel (I) können als Zwischenprodukte zur Herstellung von biologisch aktiven Verbindungen, beispielsweise von Insektiziden verwendet werden (vgl. z.B. EP-A 0 302 389).

### Herstellungsbeispiele

### Beispiel 1

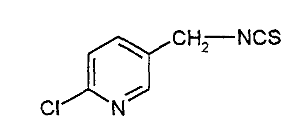

### (Zweite Stufe)

51,3 g (0,2 Mol) 2-Chlor-5-pyridylmethyl-kaliumdithiocarbamat (vgl. erste Stufe) werden in 200 ml Wasser gelöst und mit 200 ml Methylenchlorid versetzt.

Bei 0°C werden 460 ml 13 %ige Chlorlauge (NaOCl) so zugetropft, daß 5°C nicht überschritten werden. Man rührt 30 Minuten bei 0-5°C nach, trennt die organische Phase ab, extrahiert die wässrige Phase dreimal mit Methylenchlorid und trocknet die vereinten organischen Phasen mit Natriumsulfat.

Nach Abdestillieren des Lösungsmittels erhält man 31,3 g (82 % der Theorie) 2-Chlor-5-pyridylmethylisothiocyanat, das im Kühlschrank auskristallisiert (Schmelzpunkt: 22°C)

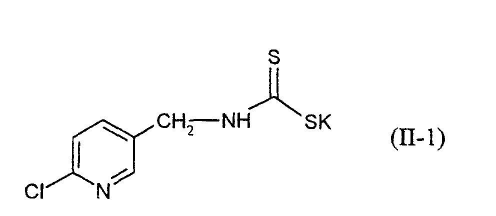

### (Erste Stufe)

2,85 g (20 mmol) 2-Chlor-5-aminomethyl-pyridin und 3,2 g (20 mmol) Kaliumethylxanthogenat werden in 30 ml Ethanol über Nacht bei Rückfluß gerührt. Man läßt abkühlen und filtriert ab: Man erhält 4,4 g (86 % der Theorie) 2-Chlor-5-pyridylmethyl-Kaliumdithiocarbamat vom Schmelzpunkt 252°C (Zers.).

## Patentansprüche

1. Verfahren zur Herstellung von Pyridylmethylisothiocyanaten der Formel (I) 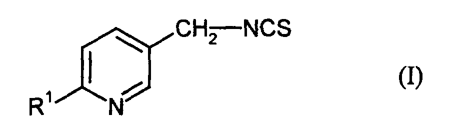 in welcher
R¹ für Halogen oder C₁-C₄-Alkyl steht,
**dadurch gekennzeichnet, dass** man
in einer ersten Stufe Amine der Formel (II) 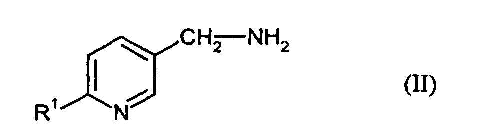 in welcher
R¹ die oben angegebene Bedeutung hat,
mit Xanthogenaten der Formel (III),
R²O―CS―S^{⊖} M^{⊕} (III)
in welcher
R² für C₁-C₄-Alkyl steht und
M für ein Alkalimetall oder für Ammonium steht,
gegebenenfalls in Gegenwart eines Verdünnungsmittels umsetzt und
in einer zweiten Stufe die so erhaltenen Dithiocarbamate der Formel (IV) 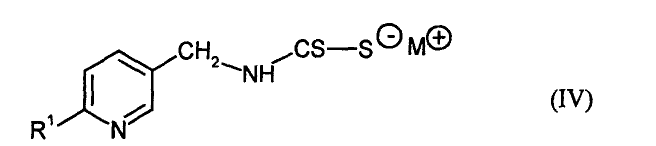 in welcher
R¹ und M die oben angegebene Bedeutung haben,
gegebenenfalls in Gegenwart eines Verdünnungsmittels zu den Isothiocyanaten der Formel (I) oxidiert.

2. Dithiocarbamate der Formel (IV) 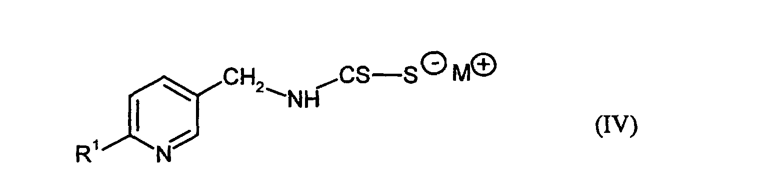 in welcher
R¹ für Halogen oder C₁-C₄-Alkyl steht und
M für ein Alkalimetall oder für Ammonium steht.

3. Verfahren zur Herstellung der Dithiocarbamate der Formel (IV) 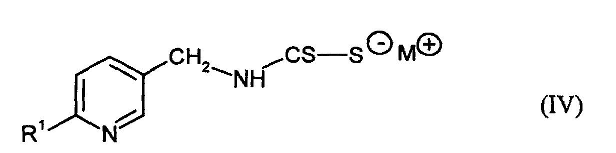 in welcher
R¹ und M die in Anspruch 1 angegebene Bedeutung haben,
**dadurch gekennzeichnet, dass** man
Amine der Formel (II) 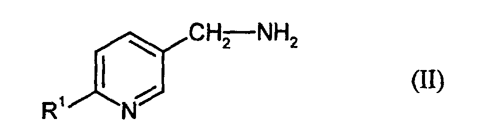 in welcher
R¹ die in Anspruch 1 angegebene Bedeutung hat,
mit Xanthogenaten der Formel (III),
R²O―CS―S^{⊖}M^{⊕} (III)
in welcher
R² für C₁-C₄-Alkyl steht und
M für ein Alkalimetall oder für Ammonium steht,
gegebenenfalls in Gegenwart eines Verdünnungsmittels umsetzt

4. Verwendung von Dithiocarbamaten der Formel (IV) 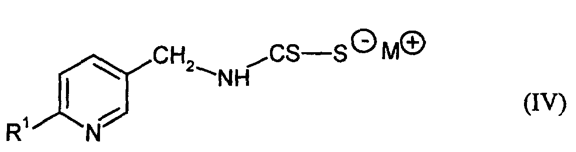 in welcher
R¹ und M die in Anspruch 2 angegebene Bedeutung haben,
zur Herstellung von Pyridylmethylisothiocyanaten der Formel (I) 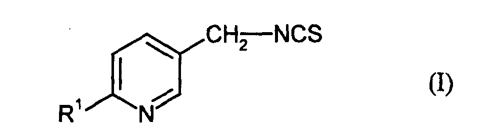 in welcher
R¹ für Halogen oder C₁-C₄-Alkyl steht,
durch Oxidation.

## Claims

1. Process for preparing pyridylmethyl isothiocyanates of the formula (I) 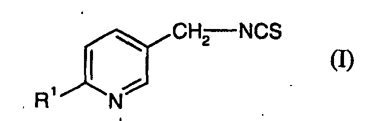 in which
R¹ represents halogen or C₁-C₄-alkyl,
**characterized in that**
in a first step, amines of the formula (II) 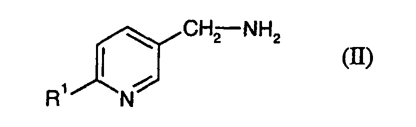 in which
R¹ is as defined above,
are reacted with xanthogenates of the formula (III)
R²O―CS―S^{⊖}M^{⊕} (III)
in which
R² represents C₁-C₄-alkyl and
M represents an alkali metal or represents ammonium,
if appropriate in the presence of a diluent, and
in a second step, the resulting dithiocarbamates of the formula (IV) 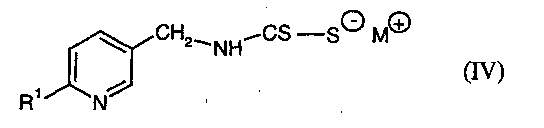 in which
R¹ and M are each as defined above,
are oxidized, if appropriate in the presence of a diluent, to give the isothiocyanates of the formula (I).

2. Dithiocarbamates of the formula (IV) 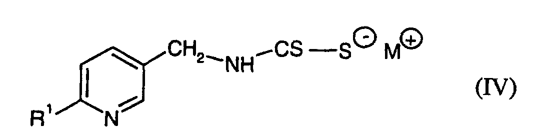 in which
R¹ represents halogen or C₁-C₄-alkyl and
M represents an alkali metal or represents ammonium.

3. Process for preparing the dithiocarbamates of the formula (IV) 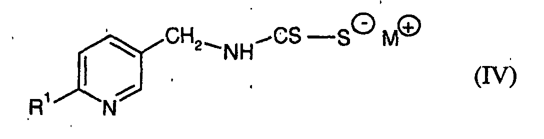 in which
R¹ and M are each as defined in Claim 1,
**characterized in that**
amines of the formula (II) 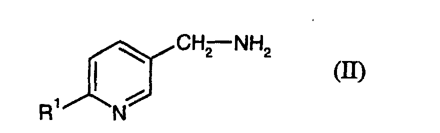 in which
R¹ is as defined in Claim 1,
are reacted with xanthogenates of the formula (III)
R²O―CS―S^{⊖}M^{⊕} (III)
in which
R² represents C₁-C₄-alkyl and
M represents an alkali metal or represents ammonium,
if appropriate in the presence of a diluent.

4. Use of dithiocarbamates of the formula (IV) 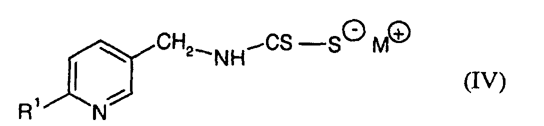 in which
R¹ and M are each as defined in Claim 2,
for preparing pyridylmethyl isothiocyanates of the formula (I) 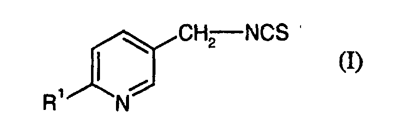 in which
R¹ represents halogen or C₁-C₄-alkyl,
by oxidation.

## Revendications

1. Procédé de production de pyridylméthylisothiocyanates de formule (I) 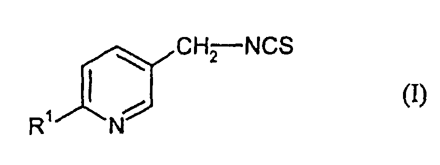 dans laquelle
R¹ est un halogène ou un groupe alkyle en C₁ à C₄,
**caractérisé en ce que**
dans une première étape, on fait réagir des amines de formule (II) 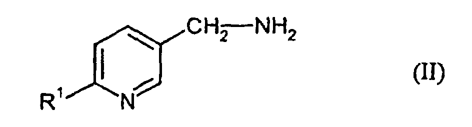 dans laquelle
R¹ a la définition indiquée ci-dessus,
avec des xanthogénates de formule (III)
R²O―CS―S^{⊖}M^{⊕} (III)
dans laquelle
R² est un groupe alkyle en C₁ à C₄ et
M est un métal alcalin ou l'ion ammonium,
le cas échéant en présence d'un diluant et
dans une seconde étape, on oxyde les dithiocarbamates ainsi obtenus de formule (IV) 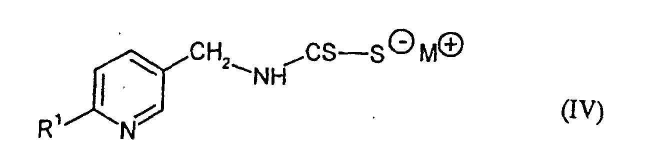 dans laquelle
R¹ et M ont la définition indiquée ci-dessus,
le cas échéant en présence d'un diluant, pour obtenir les isothiocyanates de formule (I).

2. Dithiocarbamates de formule (IV) 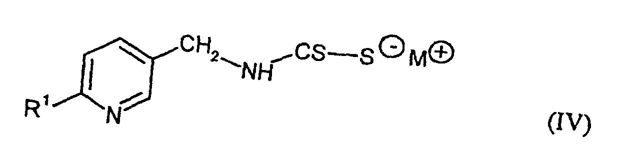 dans laquelle
R¹ est un halogène ou un groupe alkyle en C₁ à C₄ et
M est un métal alcalin ou l'ion ammonium.

3. Procédé de production de dithiocarbamates de formule (IV) 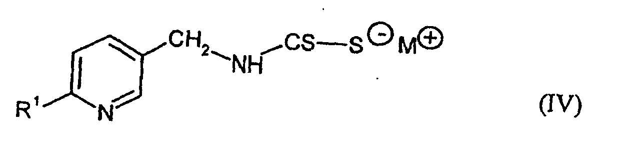 dans laquelle
R¹ et M ont la définition indiquée dans la revendication 1,
**caractérisé en ce qu'**on fait réagir des amines de formule (II) 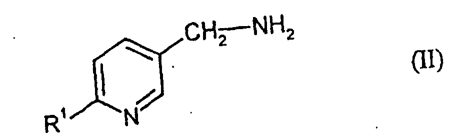 dans laquelle
R¹ a la définition indiquée dans la revendication 1,
avec des xanthogénates de formule (III)
R²O―CS―S^{⊖}M^{⊕} (III)
dans laquelle
R² est un groupe alkyle en C₁ à C₄ et
M est un métal alcalin ou un ion ammonium,
le cas échéant en présence d'un diluant.

4. Utilisation de dithiocarbamates de formule (IV) 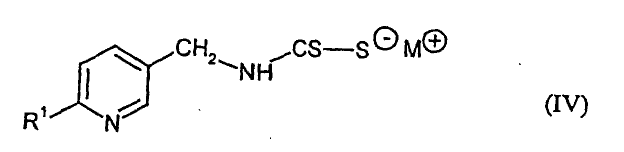 dans laquelle
R¹ et M ont la définition indiquée dans la revendication 2,
pour la production de pyridylméthylisothiocyanates de formle (I) 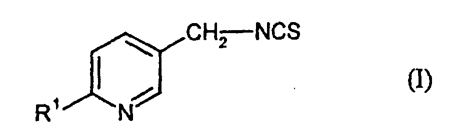 dans laquelle
R¹ est un halogène ou un groupe alkyle en C₁ à C₄,
par oxydation.
